# EUROPÄISCHE PATENTSCHRIFT

(11) **EP 3 795 194 B1**
(45) Veröffentlichungstag und Bekanntmachung des Hinweises auf die Patenterteilung: **01.11.2023**
(21) Anmeldenummer: 20200449.5
(22) Anmeldetag: 05.02.2013
(51) Int. Cl.: A61M 5/315

(54) **INJEKTIONSGERÄT ZUR VERABREICHUNG ODER FÖRDERUNG VON FLUIDEM PRODUKT**
INJECTION DEVICE FOR ADMINISTERING OR TRANSPORTING FLUID PRODUCT
APPAREIL D'INJECTION DESTINÉ À L'ADMINISTRATION OU À L'ACHEMINEMENT D'UN FLUIDE

(30) Priorität: 09.02.2012 CH 1772012
(43) Veröffentlichungstag der Anmeldung: 24.03.2021
(62) Teilanmeldung aus: 13704345.1
(73) Patentinhaber: Ypsomed AG, 3401 Burgdorf (CH)
(72) Erfinder: Burren, Stefan, 3150 Schwarzenburg (CH); Hirschel, Jürg, 3007 Bern (CH); Moser, Ulrich, 3412 Heimiswil (CH)
(74) Vertreter: Meier Obertüfer, Jürg

(56) Entgegenhaltungen:
- EP-A1- 1 294 418
- EP-B1- 1 218 042
- WO-A1-2011/068531
- US-A1- 2009 275 916

## Beschreibung

Die vorliegende Erfindung bezieht sich auf eine Injektionsvorrichtung, insbesondere auf ein Injektionsgerät mit einer Dosiervorrichtung, mit welcher eine Dosis oder Menge einer aus oder mit Hilfe des Injektionsgeräts abzugebenden Substanz eingestellt werden kann.

Aus dem Stand der Technik sind verschiedene Injektionsgeräte bekannt, insbesondere auch stift- oder penförmige Injektionsgeräte. Die WO93/07922 A1 zeigt ein solches stift- oder penförmiges Injektionsgerät. Der Pen umfasst eine Dosiervorrichtung und eine Fördereinrichtung, welche über eine zweiteilige Einwegrutschkupplung 20/21 miteinander gekoppelt sind, die Kupplung wird durch ineinander eingreifende asymmetrische Verzahnungen erreicht, wobei der Kupplungseingriff durch eine Feder unterstützt wird. Dabei weist ein Kupplungseingangsglied 20 eine axial in distale Richtung weisende Zahnung auf. Ein Kupplungsausgangsglied 21 weist eine dazu komplementäre Verzahnung auf. Die beiden Kupplungsglieder sind koaxial zur Längsachse des Pens angeordnet und relativ zum Gehäuse des Pens drehbar ausgestaltet. Die einzelnen Zähne der Verzahnungen weisen zwei Flanken auf, wobei die beiden Flanken unterschiedliche Steigungen aufweisen. Eine Flanke weist in axiale Richtung, und die zweite Flanke weist relativ zur Längsachse eine Neigung auf. Die axial ausgerichteten Flanken verhindern bei Kupplungseingriff eine Relativdrehung zwischen Kupplungseingangsglied 20 und Kupplungsausgangsglied 21 in eine Richtung, während die zweiten, geneigten Flanken eine Relativdrehung in die entgegengesetzte Richtung ermöglichen.

Zum Einstellen einer zu verabreichenden Dosis wird der Dosierknopf 14 der Dosiervorrichtung zusammen mit der Dosierhülse 15 aus dem Gehäuse des Geräts herausgeschraubt. Dabei ist das Kupplungseingangsglied 20 verdrehsicher zum Dosierknopf 14 angeordnet und das Kupplungsausgangsglied 21 verdrehsicher zur Fördereinrichtung. Beim Erhöhen der Dosis ist der Pen so ausgelegt, dass eine Relativdrehung zwischen Kupplungseingangsglied 20 und Kupplungsausgangsglied 21 stattfinden kann. Dabei gleiten die geneigten Flanke der Kupplungszahnungen über einander weg, wobei das Kupplungseingangsglied 20 axial entgegen der Federkraft bewegt wird. Beim Überschreiten der Zahnspitzen springt das Kupplungseingangsglied 20 entlang der axial ausgerichteten Flanken zurück in seine axiale Ausgangsposition und erzeugt einen hörbaren Klick. Wird der Dosierknopf 14 zurück ins Gehäuse geschraubt, so verhindert die Einwegrutschkupplung aufgrund der axial ausgerichteten Zahnflanken an den Kupplungsgliedern 20, 21 eine Relativdrehung zwischen Dosiervorrichtung und Fördereinrichtung, wobei die Rotation des Dosierknopfes somit an die Fördereinrichtung weitergegeben wird, und eine Ausschüttung von Produkt aus dem Pen stattfinden kann. Zur Dosiskorrektur muss der Kupplungseingriff zwischen den Kupplungsgliedern 20, 21 manuell und entgegen der Federkraft gelöst werden. Ist der Kupplungseingriff gelöst, so kann die Dosiervorrichtung unabhängig von der Fördereinrichtung zurück in ihre Ausgangsposition bewegt werden. Diese Art der Dosiskorrektur verlangt von der benutzenden Person Geschick und Kraft, weil beide Hände gebraucht werden um den Kupplungseingriff zu lösen und gelöst zu halten, also entgegen der Federkraft. Im gelösten Zustand muss so dann auch noch die Dosierhülse zurückgeschraubt werden. Beim Zurückschrauben der Dosierhülse entsteht bei gelöstem Kupplungseingriff auch kein Klick-Geräusch als Feedback für die benutzende Person.

EP1003581 B1 zeigt in den Figuren 15 bis 17 ebenfalls ein penförmiges Injektionsgerät. Dieses Injektionsgerät verfügt über eine Zweiwegrutschkupplung zwischen Dosiervorrichtung und Fördereinrichtung. Die Dosiervorrichtung umfasst eine Dosierhülse 80 mit einem an der Hülse angeordneten Dosierknopf 81. Die Dosierhülse 80 kann über die Buchse 82 mit dem Treiberrohr 85 der Fördereinrichtung gekoppelt werden. Die Kupplung besteht aus einem Kupplungseingangsglied (nicht gezeigt), dass an der Dosierhülse 80 fest angeordnet ist und einem Kupplungsausgangsglied 93, als Rosette bezeichnet, welches fest an der Buchse 82 angebracht ist. Die Kupplung wird durch Drücken des Ausschüttknopfes 88 in distale Richtung geschlossen und ist, solange der Ausschüttknopf nicht betätigt wird, nicht formschlüssig in Eingriff. Kupplungseingangsglied und Kupplungsausgangsglied weisen eine Zahnung auf, welche Relativrotation grundsätzlich erlauben, auch wenn die Kupplungsglieder einander aufgrund der Gewichtskräfte berühren. Solange die Kupplung nicht durch eine Betätigung des Ausschüttknopfes geschlossen wird, erlaubt dies eine Dosierbewegung zum Aufdosieren und auch zur Dosiskorrektur. In der vom Stand der Technik gezeigten Ausführung entsteht in der Zweiwegrutschkupplung kein Klickgeräusch, so dass eine Klickfunktion zusätzlich und an einem anderen Ort im Gerät angeordnet werden muss.

Die EP1218042B1 zeigt einen Injektionspen mit einer Dosiervorrichtung mit einer Dosierhülse die für die Dosiseinstellung in eine Richtung drehbar ist wobei ein akustisches Signal erzeugt wird. Für die Dosiskorrektur hat der Injektionspen ein separates reset-mechanimus wobei Dosiervorrichtung von der Fördereinrichtung entkuppelt wird.

Es ist eine Aufgabe der vorliegenden Erfindung, ein Injektionsaerät der genannten Art, d. h. eine Verabreichungsvorrichtung mit einer Dosiervorrichtung und einer Fördereinrichtung, welche über einen Kupplungsmechanismus miteinander lösbar gekoppelt werden können, die einfach aufgebaut ist, und der benutzenden Person bei der Dosiseinstellung und der Korrektur von zu hoch eingestellten Dosen ein verbessert wahrnehmbares Feedback gibt.

Diese Aufgabe wird durch den Gegenstand mit den Merkmalen des Anspruchs 1 gelöst.

Weitere, vorteilhafte Ausführungsformen ergeben sich aus den abhängigen Ansprüchen.

### Darstellung der Erfindung

Im Kontext der vorliegenden Beschreibung eines im allgemeinen penförmigen Injektionsgerätes werden verschiedene Richtungs- und Positionsangaben gemacht, die an dieser Stelle kurz erklärt werden. Mit der Orientierung axial ist Orientierung entlang der Längsachse des Injektionsgerätes gemeint. Mit distal ist dasjenige Ende des Injektionsgerätes gemeint, an welchem die Injektionsnadel angebracht wird. Proximal meint so dann korollar das entgegengesetzte Ende des Injektionsgerätes. In distale Richtung meint in die Richtung des distalen Endes schauend und in proximale Richtung dann analog in die Richtung des proximalen Endes schauend.

Die Erfindung betrifft ein verbessertes Injektionsgerät zur Verabreichung eines fluiden Produktes. Das Injektionsgerät umfasst ein Gehäuse mit einer Aufnahme für das Produkt, eine Fördereinrichtung zur Förderung des Produkts sowie eine Dosiervorrichtung für die Einstellung einer zu verabreichenden Produktdosis und zum Anzeigen der eingestellten Produktdosis. Das Gehäuse bildet eine Aufnahme für das Produkt, vorzugsweise eine Aufnahme für ein mit dem Produkt gefülltes Behältnis. Bei diesem Behältnis kann es sich vorzugsweise um eine Karpule handeln. Die Fördereinrichtung umfasst eine Kolbenstange, welche relativ zum Gehäuse in eine Förderrichtung bewegbar ist, um eine eingestellte Produktdosis in einem der eingestellten Produktdosis entsprechenden Förderhub auszuschütten. Beim Förderhub handelt es sich um eine translatorische Bewegung der Kolbenstange, bevorzugt eine lineare Schiebebewegung. In einer bevorzugten Ausführungsform wird beim Förderhub ein beweglicher Kolben des als Karpule ausgebildeten Behältnisses verschoben. Die Fördereinrichtung umfasst ferner ein Führungselement, welches die translatorische Bewegung der Kolbenstange führt. Das Führungselement ist in einer bevorzugten Ausführungsform als gehäusefeste Längsführung für die Kolbenstange ausgebildet, so dass die Kolbenstange relativ zum Führungselement axial verschoben werden kann, jedoch nicht verdreht werden kann. Ferner umfasst die Fördereinrichtung ein Antriebselement, welches mit der Kolbenstange in Eingriff steht. Das Antriebselement ist in einer bevorzugten Ausführungsform als Gewindemutter ausgebildet, deren Innengewinde mit einem entsprechenden auf der Aussenfläche der Kolbenstange angebrachten Aussengewinde in Eingriff gebracht ist. Bevorzugt ist die Gewindemutter im Gehäuse rotierbar aber axial fest angebracht. In einer möglichen bevorzugten Ausführungsform ergibt sich für den Fördermechanismus der Fördereinrichtung folgende kinematische Anordnung: eine Rotation der axial festen Gewindemutter relativ zur Kolbenstange resultiert in einer axialen Bewegung der Kolbenstange, da diese über die Längsführung relativ zum Gehäuse nicht drehen kann. In anderen ebenfalls bevorzugten Ausführungsformen kann die kinematische Anordnung auch invertiert sein. Dies über eine sogenannte kinematische Umkehrung, wobei die Gewindemutter zum Gehäuse rotativ fixiert wird und die Längsführung zum Gehäuse rotierbar und allenfalls verschiebbar gelagert wird. Wird bei dieser kinematischen Umkehrung nun die Längsführung rotiert, so schraubt sich die Kolbenstange durch das Gewinde der in diesem Falle relativ zum Gehäuse fixierten Gewindemutter. Die Dosiervorrichtung des Injektionsgerätes umfasst ein Dosiseinstellglied, bevorzugt eine Dosierhülse, welche mit der Innenseite des Gehäuses in einem Gewindeeingriff steht. Am proximalen Ende des Dosiseinstellgliedes ist ein greifbares Element angebracht, welches eine Einstellung einer gewünschten Dosis durch die benutzende Person ermöglicht. Bevorzugt vollführt das Dosiseinstellglied bei der Erhöhung einer zu verabreichenden Dosis eine Schraubbewegung aus dem Injektionsgerät heraus. Zum Verabreichen der eingestellten Dosis, resp. zur Reduktion einer allfällig zu hoch eingestellten Dosis kann das Dosiseinstellglied dann in das Injektionsgerät zurückgeschraubt werden. In einer weiteren bevorzugten Ausführungsform besteht zwischen Gehäuse und dem als Dosierhülse ausgeführten Dosiseinstellglied eine nicht selbsthemmende Gewindeverbindung, so dass die Dosierhülse durch das Aufbringen von axialen Kräften in das Injektionsgerät zurückgeschraubt werden kann.

Die Dosiervorrichtung umfasst weiter eine Kupplungsvorrichtung, welche die Dosiervorrichtung mit der Fördereinrichtung operativ verbinden kann. Die Kupplungsvorrichtung ist dabei so ausgelegt, dass das Einstellen und/oder Korrigieren einer zu verabreichenden Dosis unabhängig von der Fördereinrichtung geschehen kann und dass bei einer Verabreichung einer Dosis die Dosiervorrichtung selektiv mit der Fördereinrichtung operativ gekoppelt werden kann, so dass eine Bewegung der Dosiervorrichtung ganz oder anteilsweise an die Fördereinrichtung übertragen wird. Beispielsweise kann nur der Rotationsanteil einer Schraubbewegung einer Dosierhülse an die Fördereinrichtung übertragen werden oder alternativ nur die axiale Verschiebung. In einer Ausführungsform umfasst die Kupplungsvorrichtung eine Kupplungshülse mit einer Kupplungsfläche, wobei die Kupplungsfläche Eingriffelemente aufweist. Das als Dosierhülse ausgeführte Dosiseinstellglied weist eine Kupplungsgegenfläche auf mit Gegeneingriffselementen auf. Durch eine Kupplungsbewegung können die Kupplungsfläche und -gegenfläche miteinander in Eingriff gebracht werden und eine Relativbewegung zwischen Kupplungs- und Dosierhülse kann so unterbunden werden. In einer alternativen Ausführungsform sind Kupplungsfläche und Kupplungsgegenfläche immer in Eingriff miteinander, wobei sich die Kupplungsbewegung auf ein Blockieren von Relativbewegung zwischen Kupplungs- und Dosierhülse einschränkt. In einer weiteren bevorzugten Ausführungsform kommen Kupplungsfläche und Kupplungsgegenflächen nicht direkt aufeinander zu liegen, sondern werden durch eine sogenannte Klickscheibe voneinander getrennt. Dabei trennt die Klickscheibe die Relativbewegung von Kupplungsfläche und Kupplungsgegenfläche auf und ermöglicht so eine Optimierung der Kupplungseigenschaften. Insbesondere kann durch geschickte Formgebung in den Kupplungsflächen ein akustisches Feedbacksignal massgeschneidert werden, welches der benutzenden Person beim Dosieren und auch bei der Dosiskorrektur bzw. der Dosisänderung über Höhe der Dosis Aufschluss gibt. Die Dosiervorrichtung umfasst weiter einen Ausschüttknopf, welcher beweglich am proximalen Ende der Dosiervorrichtung gelagert ist. In einer bevorzugten Ausführungsform des Dosiseinstellglieds als Dosierhülse ist der Ausschüttknopf koaxial mit der Dosierhülse an deren proximalen Ende angebracht. Dabei ist der Knopf insbesondere zur Dosierhülse drehbar und mit einer gewissen axialen Beweglichkeit gelagert. In einer bevorzugten Ausführungsform ist auch die Kupplungshülse koaxial zur Dosierhülse angeordnet, wobei die Kupplungshülse bevorzugt zumindest teilweise innerhalb der der Dosierhülse angeordnet ist. Dabei ist in dieser Ausführungsform die Kupplungsfläche als ringförmiger Flansch auf der Aussenfläche der Hülse in deren proximalen Bereich angeordnet. Komplementär dazu ist auf der Innenseite der Dosierhülse ebenfalls ringförmig die Kupplungsgegenfläche angeordnet. Dabei sind in einer möglichen Ausführungsform die Eingriffselemente und Gegeneingriffselement axial zum Injektionsgerät orientiert, so dass in diesem Fall die Kupplungsbewegung eine Axialbewegung ist. Zum Beispiel kann der Kupplungseingriff durch ein Drücken des Ausschüttknopfes hergestellt werden. Die Anordnung von Dosierhülse, Kupplungshülse und Ausschüttknopf kann weiter auch eine Feder umfassen, welche Kupplungsfläche und Kupplungsgegenfläche in Eingriff hält. Dosierhülse und Kupplungshülse bewegen sich bei einer Dosierbewegung axial gemeinsam, wobei eine relative Verdrehung zueinander möglich ist, solange der Ausschüttknopf nicht gedrückt wird und die Kupplung als Folge nicht blockiert ist.

In einer bevorzugten Ausführungsform ist die Kupplungshülse relativ zur Gewindemutter verdrehgesichert, aber axial beweglich. Dies ermöglicht in dieser Ausführungsform eine axiale Bewegung der Kupplungshülse relativ zur Gewindemutter. Wird die Kupplung durch Druck auf den Ausschüttknopf blockiert und die Dosierhülse in die Injektionsvorrichtung eingeschraubt, so macht auch die Kupplungshülse diese Bewegung mit. Über die Verdrehsicherung zur Gewindemutter, wird ausschliesslich die Rotation auf die Gewindemutter übertragen und folglich die Kolbenstange axial bewegt.

Um sicherzustellen, dass das als Gewindemutter ausgebildete Antriebselement nur in diejenige Richtung rotieren kann, welche eine Bewegung der Kolbenstange in Ausschüttrichtung, also in die eine Ausschüttung bewirkende Richtung, zur Folge hat, ist zwischen Gehäuse und Gewindemutter bevorzugt eine sogenannte Rückdrehsicherung vorgesehen. Dabei kann es sich um eine radial gerichtete oder axial gerichtete Rückdrehsicherung handeln. Dabei ist die Rückdrehsicherung bevorzugt so ausgebildet, dass eine Drehung der Gewindemutter entgegen der Ausschüttrichtung vollständig blockiert wird. Bei Rotation in Ausschüttrichtung weist die Rückdrehsicherung bevorzugt einen gewissen Widerstand, auch Reluktanz genannt, auf, welcher überwunden muss, um eine Bewegung der Gewindemutter herbeizuführen. Dies ist deshalb vorteilhaft, um eine ungewollte Ausschüttung beim Korrigieren einer zu hoch eingestellten Dosis zu verhindern. Insbesondere sind die Rotationswiderstände der Rückdrehsicherung und der Kupplung aufeinander angepasst.

Beim Erreichen der maximal förderbaren Produktmenge wird die Axialbewegung der Kolbenstange blockiert. Dazu ist am proximalen Ende der Kolbenstange mindestens ein Anschlag angeordnet, welcher mit einem am Antriebselement angeordneten Gegenanschlag in Eingriff kommt, sobald die maximal förderbare Produktmenge aus der Injektionsvorrichtung gefördert ist. Anschlag und Gegenschlag können durch Gewindeabschlüsse radial, also senkrecht auf die Längsachse der Injektionsvorrichtung, wirken. Alternativ können Anschlag und Gegenanschlag auch axial, also parallel zur Längsachse der Injektionsvorrichtung wirken. Axiale und radiale Wirkungen können in vorteilhaften Ausführungsformen auch kombiniert sein.

### Detailliert Beschreibung der Zeichnungen

Verschiedene Ausführungsbeispiele werden nachstehend anhand von Figuren erläutert. Entsprechend wird der Fachmann erkennen, dass verschiedene Änderungen und Modifikationen an den untenstehend gezeigten Ausführungsformen vorgenommen werden können ohne vom Geist der Erfindung abzuweichen oder deren Schutzbereich zu verlassen. Die Beschreibung von wohlbekannten Funktionen und Konstruktionen wird zu Gunsten von Klarheit und Verständlichkeit bewusst kurz gehalten.

### Verzeichnis der Zeichnungen

- Figur 1: Explosionsdarstellung der Einzelteile einer ersten Ausführungsform eines erfindungsgemässen Injektionsgeräts
- Figur 2: Ansicht des Injektionsgerätes gemäss der ersten Ausführungsform
- Figur 3: Längsschnitt der ersten Ausführungsform im Ausgangszustand
- Figur 4: Längsschnitt der ersten Ausführungsform bei eingestellter Dosis
- Figur 5: Explosionsdarstellung der Einzelteile eine zweiten Ausführungsform eines erfindungsgemässen Injektionsgeräts
- Figur 6: Längsschnitt der zweiten Ausführungsform im Ausgangszustand
- Figur 7: Detailausschnitt der Kupplungsanordnung der zweiten Ausführungsform
- Figur 8: Detailausschnitt der Rückdrehsicherung der zweiten Ausführungsform
- Figur 9: Explosionsdarstellung der Einzelteile eine dritten Ausführungsform eines erfindungsgemässen Injektionsgeräts
- Figur 10: Längsschnitt der dritten Ausführungsform im Ausgangszustand
- Figur 11: Detailausschnitt der Kupplungsanordnung der dritten Ausführungsform
- Figur 12: Detailausschnitt der Rückdrehsicherung der dritten Ausführungsform
- Figur 13: Detailausschnitt der Verschnappvorrichtung für die Gewindehülse der dritten Ausführungsform

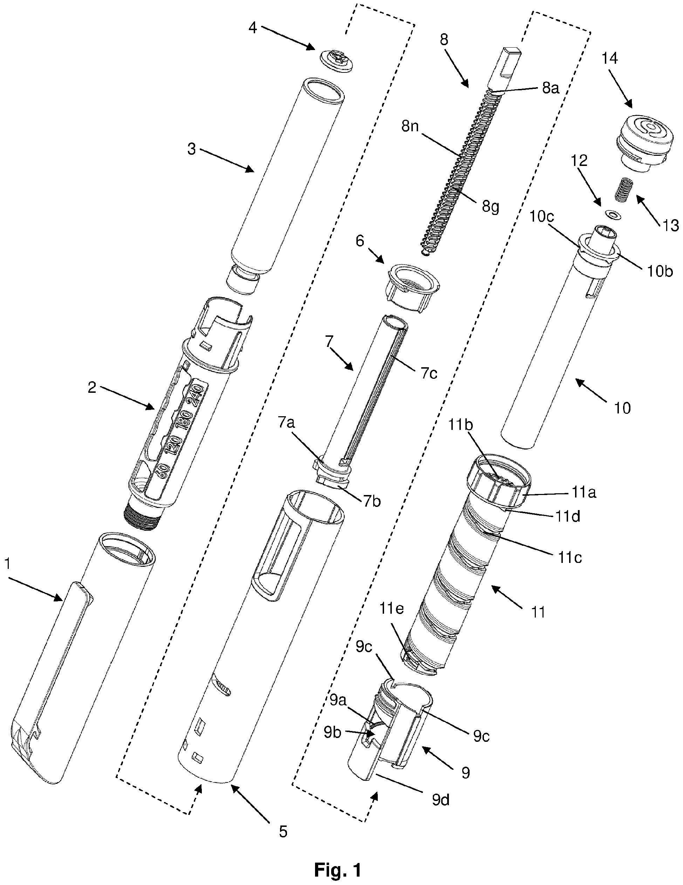

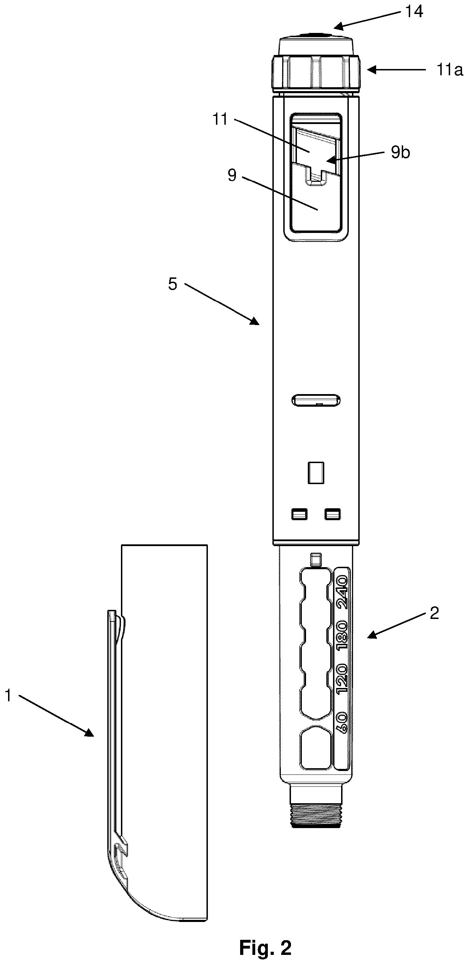

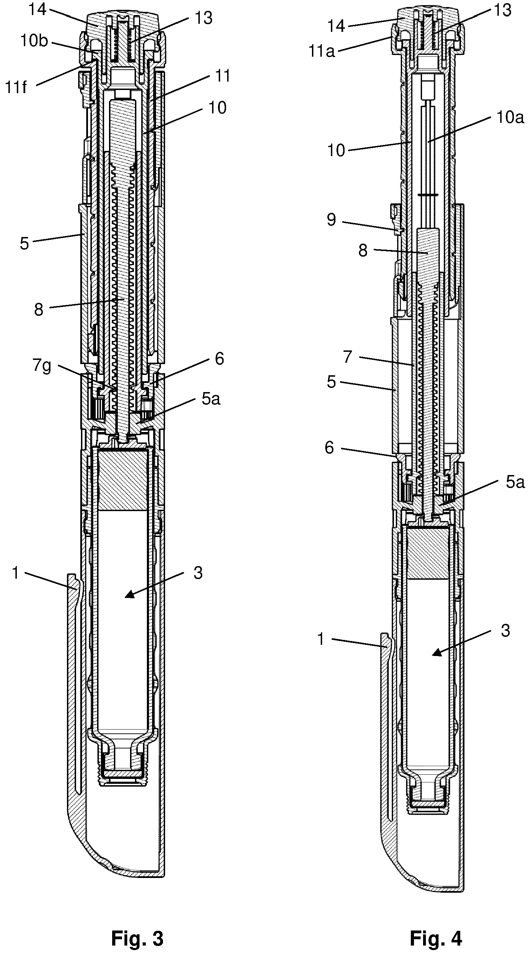

Die Figuren 1 bis 4 zeigen eine erste Ausführungsform des Injektionsgerätes. Figur 1 zeigt eine Explosionsdarstellung der Einzelteile, Figur 2 eine Darstellung der zusammengebauten Injektionsgerätes im Auslieferzustand, Figur 3 zeigt einen Längsschnitt durch das Injektionsgerät im Ausgangszustand vor dem Einstellen einer Dosis und Figur 4 zeigt denselben Längsschnitt, jedoch bei eingestellter Dosis.

Basis für das stiftförmige Injektionsgerät ist das Gehäuse 5. Über eine Schnappverbindung wird der eine Karpule 3 enthaltende Karpulenhalter 2 am Gehäuse 5 befestigt. Zumindest teilweise im Gehäuse angeordnet sind die Dosiervorrichtung und die Fördereinrichtung. In Gehäuse 5 fest eingesetzt ist die Gewindehülse 9. Gewindehülse 9 verfügt über ein Innengewinde 9a. Sinngemäss könnte die Gewindehülse auch Teil des Gehäuses sein.

Die Dosiervorrichtung umfasst die Dosierhülse 11, deren äussere Oberfläche zumindest teilweise ein Gewinde 11c trägt, welches mit dem Innengewinde 9a der Gewindehülse 9 in Eingriff ist, wobei die Gewindeverbindung zwischen Gewindehülse 9 und Dosierhülse 11 nicht selbsthemmend ausgelegt ist. Am proximalen Ende der Dosierhülse 11 ist der Drehknopf 11a angeordnet, welcher der benutzenden Person eine Dosiseinstellung ermöglicht. Die Dosierhülse 11 weist auf Ihrer Aussenfläche Markierungen in Form von Nummern auf. Beim Herausschrauben der Dosierhülse 11 aus dem Gehäuse 5 während des Dosiervorganges wird die eingestellte Dosis im Fenster 9b der Gewindehülse 9 dargestellt. Figur 3 zeigt einen Längsschnitt durch ein Injektionsgerät der ersten Ausführungsform im Ausgangszustand. Koaxial zur Dosierhülse 11 angeordnet ist die Kupplung 10. Kupplung 10 weist in ihrem proximalen Bereich einen ringförmigen Flansch 10b auf, welcher bei axialer Bewegung der Kupplung 10 relativ zur Dosierhülse 11 in distale Richtung in Eingriff mit einer dazu komplementär ausgebildeten ringförmigen Gegenfläche 11f der Dosierhülse 11 kommt. Der Flansch 10b verfügt über eine Kupplungszahnung 10c (wie in Figur 1 gezeigt), welche in Eingriff mit einer entsprechenden (Gegen-)Zahnung 11b auf der Gegenfläche 11f gebracht werden kann. Am proximalen Ende des Injektionsgeräts ist ein Ausschüttknopf 14 auf die Dosierhülse 11 so aufgeschnappt, dass der Knopf 14 sich axial relativ zur Dosierhülse 11 ein wenig bewegen kann sowie frei drehbar ist. Dabei wird die mögliche Bewegung des Ausschüttknopfes 14 durch entsprechende Führungen am proximalen Ende der Kupplung 10 geführt. Zwischen Ausschüttknopf 14 und Kupplung 10 ist die Dosierklickfeder 13 angeordnet. Im Ausgangszustand drückt die Feder 13 den Ausschüttknopf relativ zur Kupplung in proximale Richtung. Aufgrund der Verschnappung zwischen Ausschüttknopf 14 und Dosierhülse 11 wird als Reaktion die Kupplung in distale Richtung relativ zu Ausschüttknopf 14 und Dosierhülse 11 gedrückt. Insgesamt werden der Flansch 10b und die Gegenfläche 11f durch die Relaxationskraft der Feder aneinander gedrückt. Wird nun die Dosierhülse 11 relativ zur Kupplung verdreht, so rutscht die Zahnung der Gegenfläche 11f über die Zahnung des Flansches 10b. Dadurch vollführt die Kupplung 10 eine wiederholte, leichte axiale Bewegung in proximale Richtung und zurück in die Ausgangsposition. Bei geeigneter Form der Zahnung erzeugt, die Relativdrehung von Dosierhülse 11 und Kupplung 10 ein für die benutzende Person hörbares und taktil spürbares Klicken. Durch ein Drücken des Ausschüttknopfes 14 entgegen der Federkraft in distale Richtung wird eine relative Verdrehung von Kupplung 10 und Dosierhülse 11 unterbunden, so dass Kupplung 10 und Dosierhülse 11 zueinander verdrehgesichert sind.

Wie aus den Figuren 1 und 4 ersichtlich, ist die Kupplung 10 als Hülse ausgebildet. Auf Ihrer Innenfläche weist sie zwei sich gegenüberliegende und axial orientierte Führungen 10a auf, welche als Rippen ausgebildet sind. Diese Rippen greifen in entsprechende Führungen 7c, welche als axial verlaufende Nuten auf der Aussenfläche einer Gewindemutter 7 ausgebildet sind. Wie die Kupplung 10 ist die Gewindemutter 7 als Hülse ausgebildet und ist koaxial zur Kupplung im Injektionsgerät angeordnet, zumindest teilweise umgeben von der Kupplung 10. Die Gewindemutter 7 ist im Gehäuse 5 drehbar, aber axial fest angeordnet. In distale Richtung wird die Gewindemutter 7 von einer Kolbenstangenführung 5a des Gehäuses 5 gehalten, in proximaler Richtung wird die Gewindemutter 7 vom Gehäuseeinsatz 6 über den Flansch 7a gehalten. Der Gehäuseeinsatz 6 ist fest mit dem Gehäuse 5 verschnappt. Alternativ könnte er auch Teil des Gehäuses sein. Auf ihrer Innenseite verfügt die Gewindemutter 7 über ein Gewinde, welches mit dem Aussengewinde der als Gewindestange 8 ausgebildeten Kolbenstange in Eingriff steht. Die Gewindestange 8 ist relativ zum Gehäuse axial verschiebbar gelagert, jedoch durch die Kolbenstangenführung 5a verdrehgesichert. Dazu weist die Gewindestange Längsnuten 8n auf. Eine Drehung der Gewindemutter 7 relativ zur Gewindestange 8 erzwingt aufgrund der Kolbenstangenführung 5a eine Axialbewegung der Gewindestange 8 relativ zu Gewindemutter 7 und Gehäuse 5. Die Gewindemutter 7 verfügt an ihrem distalen Ende flexible Arme 7b, welche an ihrem freien Ende je einen Zahn aufweisen. Die flexiblen Arme 7b verlaufen etwa radial nach aussen, so dass die darauf angebrachten Zähne in Eingriff mit einer Rasterung (nicht gezeigt) auf der Innenseite des Gehäuses gelangen. Die flexiblen Arme 7b sind dazu in insbesondere radiale Richtung vorgespannt, sobald die Gewindemutter 7 im Gehäuse 5 eingesetzt ist. Arme, Zähne und Rasterung sind so geformt, dass eine Drehung der Gewindemutter 7 nur in eine Richtung möglich ist, wobei ein gewisser mechanischer Widerstand, die sogenannte Reluktanz, überwunden werden muss. In der in Figur 1 dargestellten Ausführung lässt sich die Gewindemutter 7 in die Richtung drehen, welche eine Bewegung der Gewindestange 8 in distale Richtung zur Folge hat. Es ist bei der vorliegenden Ausführungsform also nicht möglich die Gewindestange 8 in proximale Richtung zu bewegen. Am distalen Ender der Gewindestange 8 ist ein Flansch 4 angebracht welcher direkt auf den Stopfen der Karpule 3 wirken kann. Wird also die Gewindestange 8 in distale Richtung verschoben, so können axiale Kräfte von der Gewindestange 8 über den Flansch 4 auf den Karpulenstopfen übertragen werden, wobei eine Verschiebung des Karpulenstopfens in distale Richtung bei aufgesetzter Injektionsnadel eine Ausschüttung von Produkt zur Folge hat. Da bei der Ausschüttung die Gewindemutter relativ zum Gehäuse dreht, bewegen sich die flexiblen Arme 7b auch relativ zum Gehäuse, deren Zahnung bewegt sich über die Gehäuserasterung und erzeugt ein für die benutzende Person wahrnehmbares, akustisches, taktil spürbares Signal, welches als Ausschüttfeedback genutzt werden kann.

Im Folgenden wird die Funktion der ersten Ausführungsform des Injektionsgerätes kurz dargelegt. Die erste Ausführungsform ist als sogenannter Einweg-Pen ausgelegt. Das heisst, dass das Injektionsgerät fertig zusammengebaut, also mit zu verabreichendem Produkt, an die benutzende Person abgegeben wird. Vor Gebrauch muss die benutzende Person, das Injektionsgerät also nur entlüften, auch primen genannt. Der typische Ablauf eines Injektionsprozesses kann wie folgt aussehen: die benutzende Person entfernt die Schutzkappe 1 vom Injektionsgerät und befestigt eine Injektionsnadel (nicht gezeigt) am Nadelhalter 2a. Nun kann die Dosis über den Drehknopf 11a eingestellt werden. Dazu wird der Drehknopf 11a gedreht, so dass sich die Dosierhülse 11 aus dem Injektionsgerät herausschraubt. Die Dosierhülse wird soweit aus dem Injektionsgerät herausgeschraubt, bis die gewünschte Dosis im Fenster 9b angezeigt wird. Wird versehentlich eine zu hohe Dosis eingestellt, so kann die Dosis durch Drehen des Drehknopfes in die Gegenrichtung korrigiert werden, wodurch sich die Dosierhülse 11 zurück ins Gehäuse schraubt. Die Dosiervorrichtung beschränkt die maximal einstellbare Dosis auf einen vorgegeben Wert. Wird versucht die Dosierhülse über diesen Wert hinaus aus dem Gehäuse herauszuschrauben, so verhindern der radiale Anschlag 11e auf der Dosierhülse 11 und Gegenanschlag 9d auf der Gewindehülse 9 durch gegenseitiges Zusammenwirken eine weitere Verdrehung.

Während Dosier- und Korrekturbewegungen dreht sich die Dosierhülse relativ zur Kupplung, so dass durch die Relativbewegung der Zahnungen 10c und 11b ein Klickgeräusch entsteht. Ist die gewünschte Dosis eingestellt, so kann die Injektionsnadel am dafür vorgesehenen Ort des Körpers der benutzenden Person eingestochen werden. Dann drückt die benutzende Person den Ausschüttknopf 14 in distale axiale Richtung und blockiert so eine Relativdrehung zwischen Kupplung 10 und Dosierhülse 11. Bei weiterem Druck in distale axiale Richtung beginnt sich die Dosierhülse in einer Schraubbewegung zurück ins Gehäuse zu drehen. Aufgrund der etablierten Verdrehsicherung zwischen Dosierhülse und Kupplung, vollführt die Kupplung 10 dieselbe Bewegung wie die Dosierhülse 11. Da die Kupplung 10 permanent verdrehgesichert zur Gewindemutter 7 ist, wird die Drehbewegung der Dosierhülse 11 auf die Gewindemutter 7 übertragen. Da die Kupplung 10 jedoch axial schiebbar auf der Gewindemutter 7 gelagert ist, werden keine axialen Kräfte auf die Mutter übertragen. Wie weiter oben schon beschrieben, erzeugt die drehende Gewindemutter 7 eine axiale Bewegung der Gewindestange 8 in distale Richtung. Dabei wirkt der Flansch 4 auf den Stopfen der Karpule und verschiebt diesen, entsprechend der Verschiebung der Gewindestange 8 ebenfalls in distale Richtung, wobei die vorher eingestellte Dosis ausgeschüttet bzw. verabreicht werden kann.

Am Ende der Verabreichung, wenn die Dosierhülse vollständig ins Gehäuse zurückgeschraubt wurde, verhindern radiale Anschläge (11d, 9c) auf der Dosierhülse 11 und der Gewindehülse 9 ein Überdrehen der Dosiervorrichtung.

Wenn die letzte mögliche Menge an zu verabreichendem Produkt ausgeschüttet ist, also wenn die Karpule 3 vollständig ausgeschüttet ist, so blockiert die Fördereinrichtung eine weitere Ausschüttdrehung der Dosierhülse 11. Dabei kommt das Gewindeende 8a der Gewindestange 8 in Anschlag mit den Rippen des Innengewindes 7g der Gewindemutter 7 und verhindert eine weitere axiale Bewegung der Gewindestange 8 relativ zur Gewindemutter 7. Da die Gewindestange 8 gegenüber dem Gehäuse verdrehgesichert ist, ist auch keine gemeinsame Drehung von Gewindemutter 7 und Gewindestange 8 möglich. In Konsequenz ist ein weiteres Einschrauben der Dosierhülse 11 verhindert, solange die Verdrehsicherung zwischen Kupplung 10 und Dosierhülse 11 aufrechterhalten wird. Im Falle, dass die benutzende Person eine höhere Dosis eingestellt hat als an Produkt noch vorhanden ist, so kann im blockierten Zustand die nicht verabreichte Restmenge einfach durch Fenster 9b auf der Dosierhülse 11 abgelesen werden. Diese Restmenge kann dann in einem weiteren Verabreichungsvorgang mit einem Ersatzinjektionsgerät injiziert werden.

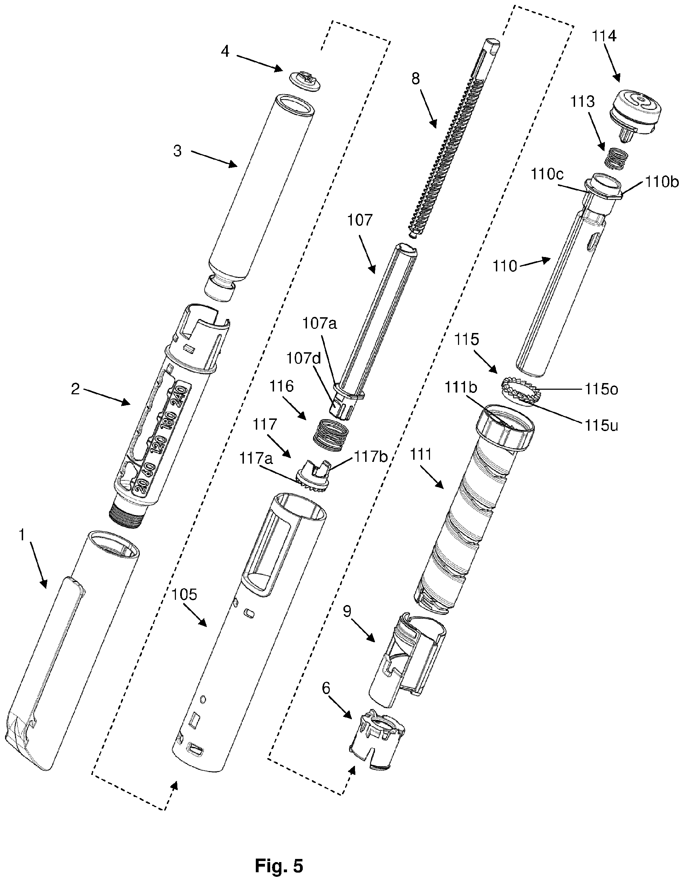

Figuren 5 bis 8 zeigen eine zweite erfindungsgemässe Ausführungsform. Diese zweite Ausführungsform ist grundsätzlich ähnlich der ersten Ausführungsform aufgebaut und funktioniert für die benutzende Person gleich. Unterschiede zeigen sich in der Rückdrehsicherung und im Kupplungsmechanismus zwischen Dosiervorrichtung und Fördereinrichtung. Zusätzlich weist die zweite Ausführungsform eine alternative Ausgestaltung des Dosierknopfs auf. Diese Änderungen werden anhand der Figuren 5 bis 8 im Folgenden dargelegt. Identische Bezeichner in den Figuren, welche sich auf mehrere Ausführungsformen beziehen, bedeuten dass die zugehörigen Elemente im wesentlichen in den verschiedenen Ausführungsformen gleich ausgestaltet sind.

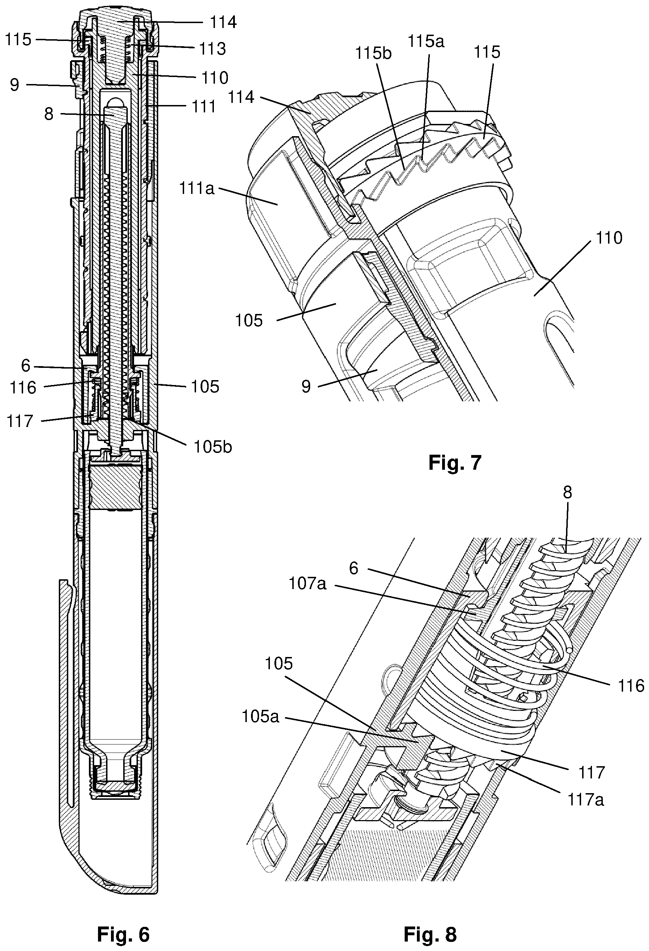

Der Kupplungsmechanismus zwischen Fördereinrichtung und Dosiervorrichtung der zweiten Ausführungsform umfasst im Vergleich zur ersten Ausführungsform ein zusätzliches Element, eine Klickscheibe 115. Die zweite Ausführungsform umfasst eine Dosierhülse 111, welche mit der Gewindehülse 9 im Gewindeeingriff ist. Weiter umfasst der Kupplungsmechanismus die Kupplung 110, welche analog der ersten Ausführungsform im Injektionsgerät angeordnet ist. Obwohl geometrisch anders ausgestaltet, wirken der Ausschüttknopf 114 und die Dosierklickfeder 113 in gleicher Weise wie in der ersten Ausführungsform. Im Unterschied zur ersten Ausführungsform greifen Flansch 110b und Gegenfläche 111f nicht direkt ineinander ein, sondern werden durch die Klickscheibe 115 voneinander getrennt. Wie in der Beschreibung zur ersten Ausführungsform schon ausgeführt, erfüllt der Kupplungsmechanismus zwei Funktionen. Die erste Funktion ist die Kupplung von Fördereinrichtung und Dosiervorrichtung und die zweite Funktion ist die Erzeugung eines akustischen Klickgeräusches bzw. einer taktil spürbaren Widerstandsänderung bei der Dosierbewegung und der Dosiskorrekturbewegung. Bei der ersten Ausführungsform sind Flansch 10b und Gegenfläche 11f als Zweiwegrutschkupplung ausgebildet, wobei die geometrischen Ausgestaltungen der Zahnungen 10c und 11b beide Drehrichtungen zu lassen müssen, was für die Erzeugung eines gut hörbaren Klickgeräusches bzw. einer taktil gut spürbaren Widerstandsänderung nachteilig sein kann. Die zweite Ausführungsform weist keine Zweiwegrutschkupplung im Sinne der ersten Ausführungsform auf. Die Drehbewegungen im Kupplungsmechanismus zwischen Kupplung 110 und Dosierhülse 111 finden örtlich getrennt statt. Zwischen Kupplungsflansch 110b und Gegenfläche 111f der Dosierhülse 111 ist koaxial die Klickscheibe 115 angeordnet (analog einer Unterlagscheibe). Die zweiseitige, eine erste und eine zweite Kupplungsfläche umfassende Klickscheibe 115 weist eine proximale Zahnung 115o auf, die in Eingriff mit der Kupplungszahnung 110c auf dem Kupplungsflansch 110b ist, und eine distale Zahnung 115u auf, die in Eingriff mit der Zahnung 111b der Gegenfläche der Dosierhülse 111 ist. Die Zahnungen 110c, 111b, 115o sowie 115u weisen bevorzugt eine asymmetrische geometrische Ausgestaltung der einzelnen Zähne auf. Figur 7 zeigt eine bevorzugte Ausgestaltung der Zahnung auf der Klickscheibe 115. In dieser Ausführungsform weist zum Beispiel die distale Zahnung 115u Zähne auf, welche eine Zahnflanke 115a aufweisen, welche in etwa in axiale Richtung ausgerichtet ist. Die Zähne 115u weisen sodann eine zweite Zahnflanke 115b, welche gegenüber der axialen Richtung geneigt ist, bevorzugt weicht der der Neigungswinkel der Zahnflanke 115 um mehr als 0°, aber um weniger als 90° von der Achse des Injektionsgeräts ab. Die Zähne der Zahnung 115o sind bevorzugt in analoger Weise ausgestaltet. Die Zähne 115o sind dabei so ausgerichtet, dass sie in einer vorteilhaften Ausgestaltung gegenüber einer Schnittebene, welche senkrecht auf die Längsachse des Injektionsgeräts steht, gespiegelt zur Zahnung 115u geformt sind. Die den Zahnungen 115o und 115u zugeordneten Zahnungen 110c, resp. 111b, der Kupplung 110, resp. der Dosierhülse 111 sind komplementär zu den Zahnungen 115 o, resp. 115u ausgestaltet. Aus der beschriebenen vorteilhaften Ausgestaltung des Kupplungsmechanismus zwischen Dosiervorrichtung und Fördereinrichtung ergibt sich eine Trennung der Gleitflächen, welche sich relativ zueinander und innerhalb des Kupplungsmechanismus bewegen während der Dosiseinstellung einerseits und der Dosiskorrektur andererseits. Aufgrund der asymmetrischen Form der Zähne 115o, 115u, 110c, 111b kann während einer Erhöhung der Dosis zwischen Kupplung 111 und der Klickscheibe 115 keine Relativbewegung stattfinden, weil die axial gerichteten Zahnflanken eine Bewegung verhindern. Umgekehrt kann bei der Erhöhung der Dosis, die Dosierhülse relativ zur Klickscheibe aufgrund der Zahnflankenorientierung bewegt werden. Bei der Dosiskorrektur ist es genau umgekehrt, das heisst, dass eine Relativbewegung zwischen Kupplung und Klickscheibe möglich ist und die Bewegung zwischen Dosierhülse und Klickscheibe verhindert wird. Die Dosierfeder 113 erzeugt eine Kraft, welche die zueinander angeordneten Zahnungen des Kupplungsmechanismus ineinander drückt. Bei einer Relativbewegung von ineinanderliegenden Zahnungen, verschieben sich die die einzelnen Zähne entlang der nicht axial gerichteten Zahnflanke und beaufschlagen die Dosierklickfeder 113 mit einer steigenden Rückstellkraft. Dieser Anstieg der Rückstellkraft ist für die benutzende Person spürbar und kann als taktile Rückmeldung dienen. Nach dem Überfahren der Zahnspitzen, springen die Zähne angetrieben durch die Rückstellkraft der Feder entlang der axial ausgerichteten Zahnflanken zurück in ihre axiale Ausgangsposition. Dabei entsteht beim Erreichen der axialen Ausgangsposition durch den Aufprall der Zahnflächen aufeinander ein akustisches Signal, auch Klick genannt. Durch die axiale Ausrichtung der einen Zahnflanke wird bei der Bewegung der Zähne in ihre axiale Ausgangsposition ein Maximum an gespeicherter Energie der Feder in die Erzeugung des Klickgeräusches verbracht und nur ein Minimum in die weitere Verschiebung der Zähne zu einander. Gegenüber der ersten Ausführungsform hat die hier gezeigte zweite Ausführungsform des Kupplungsmechanismus zwischen Dosiervorrichtung und Fördereinrichtung den Vorteil, dass mit der zweiten Ausführungsform besser wahrnehmbare akustische Feedbacksignale beim Dosieren und der Dosiskorrektur erzeugbar sind. Auch sind weitere Variationen, insbesondere auch in Verbindung mit der beschriebenen taktilen Rückmeldung, denkbar. So können die Zahnungen 115u und 115o unterschiedlich hohe Zähne bei gleicher Breite aufweisen, wobei die Zähne auf Dosierhülse 111 und der Kupplung 110 komplementär zu den zugeordneten Zähnen auf der Klickscheibe 115 ausgebildet sind. Dabei ist insbesondere die axiale Höhe der Zähne gemeint. Als Folge können Dosierklick und Dosiskorrekturklick akustisch unterscheidbar sein. In einer weiteren spannenden Ausgestaltung können die Zähne 115u und 115o eine unterschiedliche Breite aufweisen, insbesondere eine unterschiedliche Drehwinkelbreite. So kann ein Zahn 115o doppelt so breit sein wie ein Zahn 115u, so dass pro Dosiskorrekturklick eine doppelte Dosis zurückgestellt wird als mit einem Dosierklick hochgestellt wird.

Analog der ersten Ausführungsform ist die Gewindemutter 107 der zweiten Ausführungsform axial fest zum Gehäuse aber verdrehbar gelagert. Eine Rückdrehsicherung stellt zusätzlich sicher, dass die Gewindemutter nur in eine Richtung relativ zum Gehäuse rotier- oder drehbar ist. Im Vergleich zur ersten Ausführungsform ist die Rückdrehsicherung der zweiten Ausführungsform jedoch anders aufgebaut. Die Gewindemutter 107 ist an ihrem distalen Ende von einer ringförmigen Zahnscheibe 117 umgeben. Die Zahnscheibe 117 ist relativ zur Gewindemutter 107 längsverschiebbar aber verdrehgesichert gelagert sowie koaxial zur Gewindemutter angeordnet. Die Zahnscheibe 117 weist dazu axial nach proximal ragende Führungsnocken 117b auf, welche in die Führungsflächen 107d der Gewindemutter geführt sind. Die Zahnscheibe umfasst weiter eine in distale Richtung ragende Zahnung 117a welche in eine dafür vorgesehende gegen Gegenzahnung 105b der Kolbenstangenführung 105a eingreifen kann. Eine am Flansch 107a gestützte Rückdrehfeder 116 drückt die Zahnscheibe 117 in distale Richtung, so dass die Zahnung 117a mit einer definierten axialen Kraft in die Gegenzahnung 105b eingreift. Wie in Figur 8 gezeigt, ist die Zahnung 117a vorteilhaft asymmetrisch ausgestaltet, so dass eine relative Drehung der Zahnscheibe 117 zum Gehäuse 105 möglich ist und in die andere Richtung verhindert wird. Die Tatsache, dass die Zahnscheibe 117 verdrehsicher an der Gewindemutter angeordnet ist, hat zur Folge, dass auch die Gewindemutter nur in eine Richtung drehen kann. Im Vergleich zur Rückdrehsicherung der ersten Ausführungsform hat die Rückdrehsicherung der zweiten Ausführungsform den Vorteil, dass sie einfacher herzustellen ist. Ergänzend ist es auch so, dass die Rückdrehsicherung der zweiten Ausführungsform nicht zu Relaxationsphänomenen neigt. Weiter ergibt sich der Vorteil, dass durch die Einführung einer separaten Feder der Drehwiderstand der Gewindemutter bei der Drehung in die erlaubte Richtung, durch die Wahl einer passenden Feder, ohne Anpassungen des Designs variieren lässt -was bei der ersten Ausführungsform so nicht möglich ist, womit einerseits das akustische und taktile Ausschüttfeedback wie auch die Reluktanz gesteuert werden können.

Im Vergleich zur ersten Ausführungsform ist bei der zweiten Ausführungsform auch die geometrische Ausgestaltung des Ausschüttknopfes 114 geändert. Diese geometrische Änderung bleibt jedoch ohne bemerkenswerte Auswirkungen auf die Funktion.

Für die benutzende Person ergeben sich durch die Änderungen, welche in der zweiten Ausführungform gegenüber der ersten Ausführungsform vorgenommen wurden, keine grundsätzlichen Unterschiede in der Bedienung.

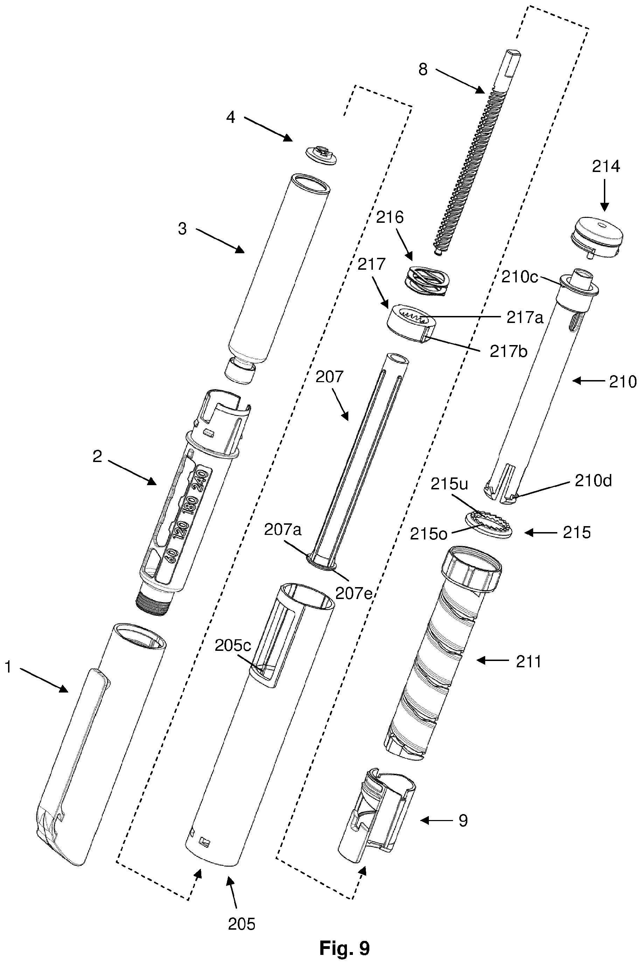

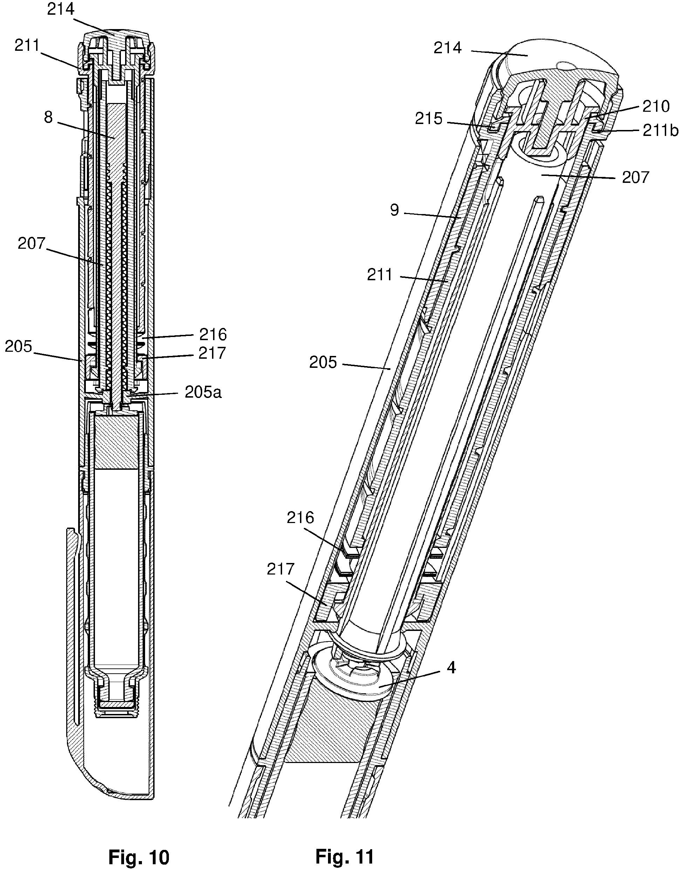

Eine dritte Ausführungsform des Injektionsgerätes wird in den Figuren 9 bis 13 dargestellt. Die dritte Ausführungssform basiert auf der zweiten Ausführungsform. Ein Vorteil der dritten Ausführungsform gegenüber der zweiten Ausführungsform ergibt sich durch die Reduktion der Anzahl der benötigten Bauteile. Ein Unterschied zwischen zweiter und dritter Ausführungsform ergibt sich aus der geänderten Anordnung der Rückdrehsicherung, welche bei der dritten Ausführungsform nicht mehr an der Gewindemutter angeordnet ist, sondern an der Kupplung, was ermöglicht, die Rückdrehfeder mit der Dosierklickfeder zu vereinen. Die spezifischen Details der dritten Ausführungsform werden im Folgenden erläutert.

Die Rückdrehsicherung der dritten Ausführungsform umfasst eine ringförmige Zahnscheibe 217 welche verschiebbar aber nicht verdrehbar im Gehäuse 205 gelagert ist. Die Lagerung passiert dabei über Führungsrippen 217b auf der Zahnscheibe 217 und Führungsnuten 205c auf der Innenseite des Gehäuses. Die Zahnscheibe 217 umfasst eine axial in distale Richtung orientierte asymmetrische Zahnung 217a auf. Die Kupplung 210 weist in der dritten Ausführungsform an ihrem distalen Ende eine axial in proximale Richtung orientierte asymmetrische Gegenzahnung 210d auf, welche in Eingriff mit der Zahnung 217a kommen kann. Wie bei der Ausgestaltung der zweiten Ausführungsform dient die asymmetrische Form der Zähne 217a und 210d dazu, Relativbewegung nur in eine Richtung zuzulassen. Dabei stellt eine Rückdrehfeder 216 sicher, dass auf die Zahnscheibe 217 eine Kraft in distale Richtung wirkt, welche nach dem Zusammenbau des Injektionsgerätes sicherstellt, dass die Zahnscheibe 217 in Eingriff mit der Zahnung 210d auf der Kupplung 210 gebracht werden kann.

In der dritten Ausführungsform wirkt die Rückdrehfeder 216 nicht nur in distale Richtung auf die Zahnscheibe 217, sondern auch auf die Dosierhülse 211 in proximale Richtung. Die Rückdrehfeder 216 ist in der gezeigten, vorteilhaften Ausgestaltung koaxial, ausserhalb der Kupplung 210 angeordnet und ist zwischen das distale Ende der Dosierhülse 211 und der Zahnscheibe 217 geklemmt. Beim Einstellen einer Dosis machen Zahnscheibe 217 und die Rückdrehfeder 216 die axiale Bewegung von Kupplung 210 und Dosierhülse 211 mit. Aus dieser Anordnung ergibt sich die zweite Wirkung der Rückdrehfeder 216. Da sie direkt die Dosierhülse 211 in proximale Richtung und indirekt die Kupplung 210 (über die Zahnscheibe 217) in distale Richtung drückt, wird dadurch auch eine Wirkung auf den Kupplungsmechanismus zwischen Dosiervorrichtung und Fördereinrichtung erzielt. Wie in der zweiten Ausführungsform sind Kupplung 210 und Dosierhülse 211 über eine Klickscheibe 215 miteinander gekuppelt. Die Klickscheibe 215 ist grundsätzlich gleich aufgebaut wie die Klickscheibe 115 und funktioniert auch gleich wie die Klickscheibe 115. Durch die Kraftwirkung der Rückdrehfeder 216 auf Kupplung 210 und Dosierhülse 211 wird die Zahnung 210c mit der Zahnung 215o in Eingriff gebracht und die Zahnung 211b mit der Zahnung 215u in Eingriff gebracht. Die zweite Wirkung der Rückdrehfeder 216 ersetzt also die Wirkung der Dosierklickfeder 113 aus der zweiten Ausführungsform, daher wird in der dritten Ausfürhungsform des Injektionsgerätes keine Dosierklickfeder mehr benötigt.

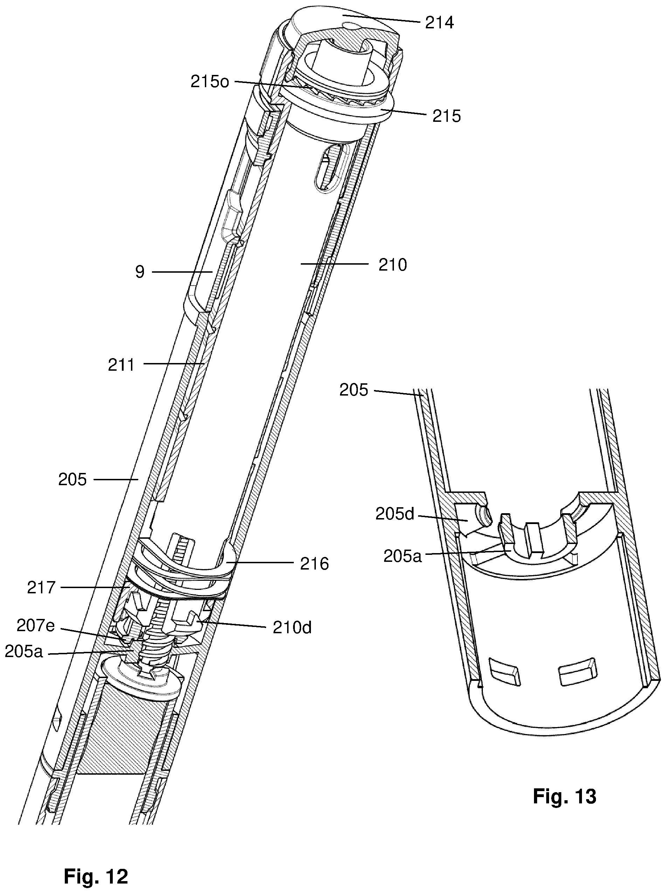

In einer weiteren Vereinfachung aus der zweiten Ausführungsform heraus kann der Gehäuseeinsatz 106 der zweiten Ausführungsform in der dritten Ausführungsform durch eine mit dem Gehäuse 205 einstückig ausgebildete Verschnappvorrichtung 205d ersetzt werden wie in Figur 13 gezeigt. Beim Zusammenbau eines Injektionsgerätes der dritten Ausführungsform wird beim Einfügen der Flansch 207a der Gewindemutter 207 in der Verschnappvorrichtung 205d des Gehäuses 205 verschnappt. Zur Vereinfachung dieses Vorgangs weist der Flansch 207a Abschrägungen 207e auf, welche das temporäre geometrische Ausweichen der Verschnappvorrichtung 205 leiten.

### Liste der Bezeichner

- 1: Schutzkappe
- 2: Karpulenhalter
- 2a: Nadelhalter
- 3: Karpule
- 4: Flansch
- 5: Gehäuse
- 5a: Kolbenstangenführung
- 6: Gehäuseeinsatz
- 7: Gewindemutter
- 7a: Flansch
- 7b: flexible Arme
- 7c: Führung
- 7g: Gewinde
- 8: Gewindestange
- 8a: Gewindeende
- 8g: Gewinde
- 8n: Längsnuten
- 9: Gewindehülse
- 9a: Innengewinde
- 9b: Fenster
- 9c: Anschlag
- 9d: Gegenanschlag
- 10: Kupplung
- 10a: Führung
- 10b: Flansch
- 10c: Kupplungszahnung
- 11: Dosierhülse
- 11a: Drehknopf
- 11b: Zahnung
- 11c: Aussengewinde
- 11d: proximale radiale Anschläge
- 11e: distaler radialer Anschlag
- 11f: Gegenfläche
- 12: Metallring
- 13: Dosierklickfeder
- 14: Ausschüttknopf

- 105: Gehäuse
- 105a: Kolbenstangenführung
- 105b: Gegenzahnung
- 107: Gewindemutter
- 107a: Flansch
- 107d: Führungsflächen
- 110: Kupplung
- 110b: Flansch
- 110c: Kupplungszahnung
- 111: Dosierhülse
- 111a: Drehknopf
- 111b: Zahnung
- 111f: Gegenfläche
- 113: Dosierklickfeder
- 114: Dosierknopf
- 115: Klickscheibe
- 115a: Zahnflanke
- 115b: Zahnflanke
- 115o: proximale Zahnung
- 115u: distale Zahnung
- 116: Rückdrehfeder
- 117: Zahnscheibe
- 117a: Zahnung/Zähne
- 117b: Führungsnocken

- 205: Gehäuse
- 205a: Kolbenstangenführung
- 205c: Längsführungsnuten
- 205d: Verschnappvorrichtung
- 207: Gewindemutter
- 207a: Flansch
- 207e: Abschrägung
- 210: Kupplung
- 210c: Kupplungszahnung
- 210d: Gegenzahnung
- 211: Dosierhülse
- 211b: Zahnung
- 214: Dosierknopf
- 215: Klickscheibe
- 215o: Zahnflanke
- 215u: Zahnflanke
- 216: Rückdrehfeder
- 217: Zahnscheibe
- 217a: Zahnung/Zähne
- 217b: Führungsrippen

## Patentansprüche

1. Ein Injektionsgerät zur Verabreichung oder Förderung von fluidem Produkt, vorzugsweise ein stiftförmiges Injektionsgerät, umfassend
- ein Gehäuse (5, 105, 205),
- eine Fördereinrichtung umfassend eine Kolbenstange (8), welche relativ zum Gehäuse (5, 105, 205) in eine Förderrichtung bewegbar ist, um eine eingestellte Produktdosis auszuschütten und ein Antriebselement (7, 107, 207), welches mit der Kolbenstange (8) in Eingriff steht
- eine Dosiervorrichtung mit einer Dosierhülse (11, 111, 211) und einem Drehknopf (11a),
- wobei der Drehknopf (11a) während des Dosiervorganges in eine Richtung und während der Dosiskorrektur in die Gegenrichtung gedreht wird,
- einen koaxial auf der Dosierhülse (11, 11, 211) angeordneten Ausschüttknopf (14, 114, 214) und
- eine Aufnahme (2) für das Produkt,
wobei die Dosiervorrichtung über einen Kupplungsmechanismus kuppelbar mit der Fördereinrichtung verbunden ist, wobei
- der Kupplungsmechanismus aus mindestens zwei jeweils eine Zahnung (10c, 110c, 115o, 115u, 210c, 11b, 111b) aus Zähnen tragenden Kupplungsflächen (10b, 110b, 11f) besteht, und wobei
- die mindestens zwei Kupplungsflächen (10b, 110b, 11f) mittels einer Feder (13, 113, 216) in Eingriff bringbar sind, wobei
- die Kupplungsflächen (10b, 11f) während des Dosiervorganges und während Dosiskorrektur dazu dienen mittels einer Relativbewegung, ein akustisches und/oder taktiles Signal entsprechend der eingestellten oder korrigierten Dosis des zu verabreichenden Produkts zu erzeugen und
- wobei die Relativbewegung zwischen den mindestens zwei Kupplungsflächen (10b, 110b, 11f) durch ein Auslösen des Ausschüttknopfs (14, 114, 214) verhindert werden kann,
**dadurch gekennzeichnet, dass**
- der Kupplungsmechanismus ein Kupplungsglied (10, 110, 210) aufweist, welches verdrehsicher aber axial verschiebbar am Antriebselement (7, 107, 207) angeordnet ist,
- am Kupplungsglied (10, 110, 210) eine der mindestens zwei Kupplungsflächen (10b, 110b,11f) fest angebracht ist, und
- an der Dosierhülse (11, 111, 211) eine weitere der mindestens zwei Kupplungsflächen (10b, 110b, 11f) fest angebracht ist, so dass die Kupplungsfläche (10b, 110b des Kupplungsglieds (10, 110, 210) und die Kupplungsfläche (11f) der Dosierhülse (11, 111, 211) direkt oder indirekt miteinander in einen Kupplungseingriff bringbar sind,
wobei zwischen Gehäuse (5, 105, 205) und Antriebselement (7, 107, 207) eine Rückdrehsicherung (7b, 117) angeordnet ist, so dass das Antriebselement (7, 107, 207) nur in Ausschüttrichtung drehen kann und eine Drehung des Antriebselements (7, 107, 207) entgegen der Ausschüttrichtung blockiert wird.

2. Ein Injektionsgerät nach Anspruch 1, wobei es sich um eine radial gerichtete oder axial gerichtete Rückdrehsicherung (7b, 117) handelt.

3. Ein Injektionsgerät nach Anspruch 1 oder 2, wobei das Antriebselement als Gewindemutter (7, 107) ausgebildet ist.

4. Ein Injektionsgerät nach Anspruch 3, wobei die Gewindemutter (7) an ihrem distalen Ende flexible Arme (7b) aufweist, welche radial nach aussen verlaufen und an ihrem freien Ende je einen Zahn aufweisen, welche in Eingriff mit einer Rasterung auf einer Innenseite des Gehäuses (5) bringbar sind um die Rückdrehsicherung zu bilden.

5. Ein Injektionsgerät nach Anspruch 3, wobei das Injektionsgerät eine ringförmige Zahnscheibe (117) umfasst, welche relativ zur Gewindemutter (107) längsverschiebbar aber verdrehgesichert gelagert sowie koaxial zur Gewindemutter (107) angeordnet ist, wobei die Zahnscheibe (117) eine in distale Richtung ragende Zahnung (117a) umfasst, welche in eine Gegenzahnung (105b) eingreifen kann um die Rückdrehsicherung zu bilden.

6. Ein Injektionsgerät nach Anspruch 5, wobei die Zahnung (117a) asymmetrisch ausgestaltet, so dass eine relative Drehung der Zahnscheibe (117) zum Gehäuse (105) möglich ist und in die andere Richtung verhindert wird.

7. Ein Injektionsgerät nach Anspruch 5 oder 6, wobei das Injektionsgerät weiter eine Rückdrehfeder (116) umfasst, welche die Zahnscheibe (117) in distale Richtung drückt, so dass die Zahnung (117a) mit einer definierten axialen Kraft in die Gegenzahnung (105b) eingreift.

8. Ein Injektionsgerät nach einem der Ansprüche 5 bis 7, wobei die Zahnscheibe (117) an einem distalen Ende der Gewindemutter (107) angeordnet ist.

9. Ein Injektionsgerät nach einem der Ansprüche 5 bis 8, wobei die Zahnscheibe (117) axial nach proximal ragende Führungsnocken (117b) aufweist, welche in Führungsflächen (107d) der Gewindemutter (107) geführt sind.

10. Ein Injektionsgerät nach einem der Ansprüche 5 bis 9, wobei die Gegenzahnung (105b) im Gehäuse (105) ausgebildet ist, insbesondere in einer Kolbenstangenführung (105a) des Gehäuses (105).

11. Ein Injektionsgerät nach einem der Ansprüche 3 bis 10, wobei die Gewindemutter (7, 107, 207) ein Innengewinde aufweist, welches mit einem auf einer Aussenfläche der Kolbenstange (8) angeordneten Aussengewinde in Eingriff gebracht werden kann und wobei die Kolbenstange (8) im Gehäuse (5, 105, 205) nicht drehbar gelagert ist.

12. Ein Injektionsgerät nach einem der Ansprüche 3 bis 10, wobei die Gewindemutter (7, 107, 207) zum Gehäuse (5,105, 205) rotativ fixiert ist und eine Längsführung für die Kolbenstange zum Gehäuse (5,105, 205) rotierbar gelagert ist, so dass sich bei Rotation der Längsführung die Kolbenstange (8) durch das Gewinde der fixierten Gewindemutter (5, 105, 205) schraubt.

13. Ein Injektionsgerät nach einem der Ansprüche 1 bis 12, wobei die Zahnungen (10c, 11b, 111b, 110c, 210c,) aus einzelnen Zähnen bestehen.

14. Ein Injektionsgerät nach einem der Ansprüche 1 bis 13, wobei der Kupplungseingriff indirekt ist und wobei
- eine Klickscheibe (115), welche eine erste und eine zweite Kupplungsfläche aufweist, zwischen den Kupplungsflächen (110b, 111f) des Kupplungsgliedes und der Dosierhülse (111) angeordnet ist,
- wobei die erste Kupplungsfläche der Klickscheibe (115) eine Zahnung (115o) aufweist, welche komplementär zur Zahnung (110c) auf dem Kupplungsglied (110) ausgebildet ist und
- die zweite Kupplungsfläche der Klickscheibe (115) komplementär zur Zahnung (111f) auf der Dosierhülse (111) ausgebildet ist.

15. Ein Injektionsgerät nach Anspruch 14, wobei die Zähne der Zahnungen (110c, 111b) eine asymmetrische Form aufweisen, so dass bei nicht ausgelöstem Ausschüttknopf (114) eine Relativbewegung in eine Richtung zwischen Dosierhülse (111) und Klickscheibe (115) stattfinden kann und in die entgegengesetzte Richtung eine Relativbewegung zwischen Kupplungsglied (110) und Klickscheibe (115) stattfinden kann.

## Claims

1. An injection device for administering or delivering fluid product, preferably a pen-shaped injection device, comprising
- a housing (5, 105, 205),
- a delivery device comprising a piston rod (8), which is movable relative to the housing (5, 105, 205) in a delivery direction in order to dispense a set product dose, and a drive element (7, 107, 207), which is in engagement with the piston rod (8),
- a dosing apparatus having a dosing sleeve (11, 111, 211) and a rotary knob (11a),
- the rotary knob (11a) being rotated in one direction during the dosing process and in the opposite direction during the dose correction,
- a dispensing button (14, 114, 214) arranged coaxially on the dosing sleeve (11, 11, 211) and
- a receptacle (2) for the product,
the dosing apparatus being able to be coupled to the delivery device via a coupling mechanism,
- the coupling mechanism consisting of at least two coupling surfaces (10b, 11 0b, 11f) each having a toothing (10c, 110c, 115o, 115u, 210c, 11b, 111b) consisting of teeth, and
- the at least two coupling surfaces (10b, 110b, 11f) being able to be brought into engagement by means of a spring (13, 113, 216),
- the coupling surfaces (10b, 11f) during the dosing process and during dose correction serving to generate an acoustic and/or tactile signal according to the set or corrected dose of the product to be administered, and
- wherein the relative movement between the at least two coupling surfaces (10b, 110b, 11f) being able to be prevented by triggering the dispensing button (14, 114, 214),
**characterized in that**
- the coupling mechanism has a coupling member (10, 110, 210), which is arranged non-rotatable but axially displaceable on the drive element (7, 107, 207),
- one of the at least two coupling surfaces (10b, 110b, 11f) is rigidly attached to the coupling member (10, 110, 210), and
- a further one of the at least two coupling surfaces (10b, 110b, 11f) is rigidly attached to the dosing sleeve (11, 111, 211), so that the coupling surface (10b, 110b of the coupling member (10, 110, 210) and the coupling surface (11f) of the dosing sleeve (11, 111, 211) can be brought directly or indirectly into a coupling engagement with one another,
a back drive safety (7b, 117) being arranged between the housing (5, 105, 205) and the drive element (7, 107, 207), so that the drive element (7, 107, 207) can rotate only in the dispensing direction and a rotation of the drive element (7, 107, 207) counter to the dispensing direction will be blocked.

2. An injection device according to claim 1, wherein the back drive safety (7b, 117) is a radially directed or axially directed anti-rotation means.

3. An injection device according to either claim 1 or claim 2, wherein the drive element is provided as a threaded nut (7, 107).

4. An injection device according to claim 3, wherein the threaded nut (7) has, at its distal end, flexible arms (7b) which extend radially outwards and each have a tooth at the free end thereof, which tooth can be brought into engagement with a catch on an inner side of the housing (5) in order to form the back drive safety.

5. An injection device according to claim 3, wherein the injection device comprises an annular toothed disk (117) which is mounted in a longitudinally displaceable but non-rotatable manner relative to the threaded nut (107) and is arranged coaxially to the threaded nut (107), wherein the toothed disk (117) comprises a toothing (117a) projecting in the distal direction, which toothing can engage in a counter toothing (105b) in order to form the back drive safety.

6. An injection device according to claim 5, wherein the toothing (117a) is asymmetrical, so that a rotation of the toothed disk (117) relative to the housing (105) is possible and is prevented in the other direction.

7. An injection device according to either claim 5 or claim 6, wherein the injection device further comprises a return spring (116) which presses the toothed disk (117) in the distal direction, so that the toothing (117a) engages in the counter toothing (105b) with a defined axial force.

8. An injection device according to any of claims 5 to 7, wherein the toothed disk (117) is arranged at a distal end of the threaded nut (107).

9. An injection device according to any of claims 5 to 8, wherein the toothed disk (117) has axially proximally projecting guide cams (117b) which are guided in guide surfaces (107d) of the threaded nut (107).

10. An injection device according to any of claims 5 to 9, wherein the counter toothing (105b) is formed in the housing (105), in particular in a piston rod guide (105a) of the housing (105).

11. An injection device according to any of claims 3 to 10, wherein the threaded nut (7, 107, 207) has an internal thread which can be brought into engagement with an external thread arranged on an outer surface of the piston rod (8) and wherein the piston rod (8) is non-rotatably mounted in the housing (5, 105, 205).

12. An injection device according to any of claims 3 to 10, wherein the threaded nut (7, 107, 207) is rotationally fixed to the housing (5, 105, 205) and a longitudinal guide for the piston rod is mounted so as to be rotatable relative to the housing (5, 105, 205), so that when the longitudinal guide rotates, the piston rod (8) screws through the thread of the fixed threaded nut (5, 105, 205).

13. An injection device according to any of claims 1 to 12, wherein the toothings (10c, 11b, 111b, 110c, 210c) consist of individual teeth.

14. An injection device according to any of claims 1 to 13, wherein the coupling engagement is indirect and wherein
- a click disk (115), which has a first and a second coupling surface, is arranged between the coupling surfaces (110b, 111f) of the coupling member and the dosing sleeve (111),
- wherein the first coupling surface of the click disk (115) has a toothing (115o) which is complementary to the toothing (110c) on the coupling member (110) and
- the second coupling surface of the click disk (115) is complementary to the toothing (111f) on the dosing sleeve (111).

15. An injection device according to claim 14, wherein the teeth of the toothings (110c, 111b) have an asymmetrical shape, so that when the dispensing button (114) is not released, a relative movement between the dosing sleeve (111) and the click disk (115) can take place in one direction and a relative movement between the coupling member (110) and the click disk (115) can take place in the opposite direction.

## Revendications

1. Appareil d'injection pour l'administration ou le transport d'un produit fluide, de préférence appareil d'injection en forme de stylo, comprenant
- un boîtier (5, 105, 205),
- une installation de transport comprenant une tige de piston (8), laquelle peut être déplacée dans un sens de transport par rapport au boîtier (5, 105, 205), pour distribuer une dose de produit réglée, et un élément d'entraînement (7, 107, 207), lequel est en prise avec la tige de piston (8)
- un dispositif de dosage comportant un manchon de dosage (11, 111, 211) et un bouton rotatif (11a),
- dans lequel le bouton rotatif (11a) est tourné dans un sens pendant le processus de dosage et dans le sens opposé pendant la correction de dose,
- un bouton de distribution (14, 114, 214) disposé de manière coaxiale sur le manchon de dosage (11, 11, 211) et
- un logement (2) pour le produit,
dans lequel le dispositif de dosage est relié de manière à pouvoir être accouplé à l'installation de transport par l'intermédiaire d'un mécanisme d'accouplement, dans lequel
- le mécanisme d'accouplement est constitué d'au moins deux surfaces d'accouplement (10b, 110b, 11f) portant respectivement une denture (10c, 110c, 115o, 115u, 210c, 11b, 111b) constituée de dents, et dans lequel
- les au moins deux surfaces d'accouplement (10b, 110b, 11f) peuvent être amenées en prise au moyen d'un ressort (13, 113, 216), dans lequel
- les surfaces d'accouplement (10b, 11f), pendant le processus de dosage et pendant la correction de dose, servent, au moyen d'un mouvement relatif, à produire un signal acoustique et/ou tactile correspondant à la dose réglée ou corrigée du produit à administrer et
- dans lequel le mouvement relatif entre les au moins deux surfaces d'accouplement (10b, 110b, 11f) peut être empêché par un déclenchement du bouton de distribution (14, 114, 214),
**caractérisé en ce que**
- le mécanisme d'accouplement présente un organe d'accouplement (10, 110, 210) qui est disposé sur l'élément d'entraînement (7, 107, 207) de manière à ne pas pouvoir tourner mais à pouvoir coulisser axialement,
- une des au moins deux surfaces d'accouplement (10b, 110b, 11f) est montée de manière fixe sur l'organe d'accouplement (10, 110, 210), et
- une autre des au moins deux surfaces d'accouplement (10b, 110b, 11f) est montée de manière fixe sur le manchon de dosage (11, 111, 211), de sorte que la surface d'accouplement (10b, 110b de l'organe d'accouplement (10, 110, 210) et la surface d'accouplement (11f) du manchon de dosage (11, 111, 211) peuvent être amenées en prise d'accouplement directement ou indirectement l'une avec l'autre,
dans lequel une sécurité anti-retour (7b, 117) est disposée entre le boîtier (5, 105, 205) et l'élément d'entraînement (7, 107, 207), de sorte que l'élément d'entraînement (7, 107, 207) ne peut tourner que dans le sens de distribution et une rotation de l'élément d'entraînement (7, 107, 207) à l'encontre du sens de distribution est bloquée.

2. Appareil d'injection selon la revendication 1, dans lequel celui-ci est une sécurité anti-retour (7b, 117) dirigée radialement ou dirigée axialement.

3. Appareil d'injection selon la revendication 1 ou 2, dans lequel l'élément d'entraînement est réalisé en tant qu'écrou fileté (7, 107).

4. Appareil d'injection selon la revendication 3, dans lequel l'écrou fileté (7) présente, au niveau de son extrémité distale, des bras flexibles (7b) qui s'étendent radialement vers l'extérieur et présentent respectivement une dent au niveau de leur extrémité libre, lesquelles dents peuvent être amenées en prise avec un encliquetage sur un côté intérieur du boîtier (5) afin de former la sécurité anti-retour.

5. Appareil d'injection selon la revendication 3, dans lequel l'appareil d'injection comprend un disque denté (117) annulaire qui est monté de manière à pouvoir coulisser longitudinalement mais de manière bloquée en rotation par rapport à l'écrou fileté (107) et est disposé de manière coaxiale par rapport à l'écrou fileté (107), dans lequel le disque denté (117) comprend une denture (117a) faisant saillie dans la direction distale et pouvant venir en prise dans une contre-denture (105b) afin de former la sécurité anti-retour.

6. Appareil d'injection selon la revendication 5, dans lequel la denture (117a) est conçue de manière asymétrique de sorte qu'une rotation relative du disque denté (117) par rapport au boîtier (105) est permise et est empêchée dans l'autre sens.

7. Appareil d'injection selon la revendication 5 ou 6, dans lequel l'appareil d'injection comprend en outre un ressort anti-retour (116) poussant le disque denté (117) dans une direction distale de sorte que la denture (117a) vient en prise dans la contre-denture (105b) avec une force axiale définie.

8. Appareil d'injection selon l'une des revendications 5 à 7, dans lequel le disque denté (117) est disposé au niveau d'une extrémité distale de l'écrou fileté (107).

9. Appareil d'injection selon l'une des revendications 5 à 8, dans lequel le disque denté (117) présente des cames de guidage (117b) faisant saillie axialement vers le côté proximal et guidées dans des surfaces de guidage (107d) de l'écrou fileté (107).

10. Appareil d'injection selon l'une des revendications 5 à 9, dans lequel la contre-denture (105b) est réalisée dans le boîtier (105), en particulier dans un guidage de tige de piston (105a) du boîtier (105).

11. Appareil d'injection selon l'une des revendications 3 à 10, dans lequel l'écrou fileté (7, 107, 207) présente un filetage interne qui peut être amené en prise avec un filetage externe disposé sur une surface externe de la tige de piston (8) et dans lequel la tige de piston (8) est montée de manière à ne pas pouvoir tourner dans le boîtier (5, 105, 205).

12. Appareil d'injection selon l'une des revendications 3 à 10, dans lequel l'écrou fileté (7, 107, 207) est fixé de manière rotative par rapport au boîtier (5, 105, 205) et un guide longitudinal pour la tige de piston est monté de manière à pouvoir pivoter par rapport au boîtier (5, 105, 205), de sorte que, lors de la rotation du guide longitudinal, la tige de piston (8) se visse à travers le filetage de l'écrou fileté (5, 105, 205) fixé.

13. Appareil d'injection selon l'une des revendications 1 à 12, dans lequel les dentures (10c, 11b, 111b, 110c, 210c,) sont constituées de dents individuelles.

14. Appareil d'injection selon l'une des revendications 1 à 13, dans lequel la prise d'accouplement est indirecte et dans lequel
- un disque d'encliquetage (115) qui présente une première et une seconde surface d'accouplement est disposé entre les surfaces d'accouplement (110b, 111f) de l'organe d'accouplement et du manchon de dosage (111),
- dans lequel la première surface d'accouplement du disque d'encliquetage (115) présente une denture (115o) complémentaire de la denture (110c) sur l'organe d'accouplement (110) et
- la seconde surface d'accouplement du disque d'encliquetage (115) est complémentaire de la denture (111f) sur le manchon de dosage (111).

15. Appareil d'injection selon la revendication 14, dans lequel les dents des dentures (110c, 111b) présentent une forme asymétrique, de sorte que, lorsque le bouton de distribution (114) n'est pas déclenché, un mouvement relatif peut avoir lieu dans un sens entre le manchon de dosage (111) et le disque d'encliquetage (115) et un mouvement relatif entre l'organe d'accouplement (110) et le disque d'encliquetage (115) peut avoir lieu dans le sens opposé.
